(19)

Europäisches Patentamt
European Patent Office
Office européen des brevets

(11) **EP 4 454 576 A1**

(12) **EUROPEAN PATENT APPLICATION**
published in accordance with Art. 153(4) EPC

(43) Date of publication:
**30.10.2024 Bulletin 2024/44**

(21) Application number: **22910192.8**

(22) Date of filing: **23.12.2022**

(51) International Patent Classification (IPC):
***A61B 17/32*** (2006.01)　　***A61B 18/12*** (2006.01)

(86) International application number:
**PCT/CN2022/141414**

(87) International publication number:
**WO 2023/116881 (29.06.2023 Gazette 2023/26)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **24.12.2021 CN 202111595350**

(71) Applicants:
• **Shanghai Yichao Medical Devices Co., Ltd.**
**Shanghai 201201 (CN)**
• **Qingdao Medbios Medical Technology Co., Ltd**
**Qingdao, Shandong 266114 (CN)**

(72) Inventors:
• **XU, Wangyang**
**Qingdao, Shandong 266114 (CN)**
• **FENG, Qingyu**
**Qingdao, Shandong 266114 (CN)**
• **SHI, Yiping**
**Qingdao, Shandong 266114 (CN)**
• **ZHAO, Dongdong**
**Qingdao, Shandong 266114 (CN)**

(74) Representative: **Petraz, Gilberto Luigi et al
GLP S.r.l.
Viale Europa Unita, 171
33100 Udine (IT)**

(54) **DEVICE FOR OUTPUTTING DRIVE SIGNAL TO SURGICAL INSTRUMENT**

(57)　A device for outputting a drive signal to a surgical instrument, comprising a housing. The housing is internally provided with: a power supply module, comprising a power supply module (121) and a power supply circuit board (122) for providing a power supply; a main board (123); an ultrasonic energy circuit board (124) connected to the main board (123) for generating an ultrasonic drive signal; and a high-frequency electric energy circuit board connected to the main board (123) for generating a high-frequency electric drive signal, wherein the projection of the ultrasonic energy circuit board (124) and the projection of the high-frequency electric energy circuit board on the bottom surface of the housing at least partially overlap, and a shielding unit is provided outside the ultrasonic energy circuit board (124) and/or the high-frequency electric energy circuit board. According to the technical solution, the interference of the high-frequency electric energy circuit board on the ultrasonic energy circuit board is effectively controlled, so that both the circuit boards can be packaged in one output device; the device can simultaneously drive an ultrasonic surgical instrument and a high-frequency electrosurgical instrument, thereby saving a device space, reducing costs, and a facilitating use of user.

FIG. 5

## Description

### Field of the Invention

The present disclosure relates to the field of ultrasonic electrosurgical systems for performing surgical operations , and in particular device for output drive signals to surgical device.

### Background of the Invention

Both the ultrasonic surgical instrument (referred to ultrasonic scalpel) and the high-frequency electrosurgical instrument (referred to electric scalpel) can be used for surgery. The ultrasonic scalpel has good cutting performance, but has poor coagulation performance in the surgical operation. The electric scalpel is divided into the monopolar electric scalpel and the bipolar electric scalpel according to the working mode. The bipolar electric scalpel has good coagulation performance, but has poor cutting performance in the surgical operation. After the electrode is arranged on the end effector of the ultrasonic scalpel, the effects of the ultrasonic scalpel and the bipolar electric scalpel can be achieved, and such multifunctional ultrasonic surgical instrument is referred to as "ultrasonic-electric scalpel".

### Summary of The Invention

### Technical Problems

In a surgical operation, the ultrasonic-electric scalpel, the ultrasonic scalpel, the monopolar electric scalpel, or the bipolar electric scalpel can be used according to needs, and several surgical instruments therein are often used in one surgery. At the present stage, the output devices for driving these surgical instruments are independent devices, and these output devices not only occupy a limited device space of an operating room, but also have a lot of inconvenience in controlling these output devices when they are used, and even affect the operation effect. For example, when an ultrasonic scalpel is driven by a separate ultrasonic output device, and a monopolar electric scalpel or a bipolar electric scalpel is driven by a separate high-frequency electric output device, and when the ultrasonic scalpel is driven, there is a usage mode in which an ultrasonic output device and a high-frequency electric output device are connected via a cable, there is also a method of driving by a device having two energy sources, but an ultrasonic drive signal and a high-frequency electric drive signal output by the device are superimposed single-channel signals, a special ultrasonic transducer is required to separate signals so as to drive the ultrasonic scalpel, and the ultrasonic transducer is a vulnerable part, which will undoubtedly increase the application cost.

### Solutions to the Problem

### Technical Solutions

In order to solve the problems in the related art, an embodiment of the present disclosure provide an apparatus for outputting drive signals to surgical instrument.
One aspect of the present disclosure provides device for outputting drive signals to surgical instrument, comprising a housing, wherein the housing is internally provided with a power supply module comprising a power supply module and a power supply circuit board, and the power supply module is configured to provide a power supply; a main board; an ultrasonic energy circuit board connected to the main board and used for generating an ultrasonic drive signal; and a high-frequency electric energy circuit board connected to the main board and used for generating a high-frequency electric drive signal; wherein projections of the ultrasonic energy circuit board and the high-frequency energy circuit board on a bottom face of the housing at least partially overlap, and a shielding unit is provided outside the ultrasonic energy circuit board and/or the high-frequency energy circuit board.
According to an embodiment of the present disclosure, the ultrasonic energy circuit board is provided with an ultrasonic frequency regulation module and an ultrasonic power regulation module.
According to an embodiment of the present disclosure, the high-frequency electric energy circuit board is provided with a high-frequency electric frequency regulation module and a high-frequency electric power regulation module.
According to an embodiment of the present disclosure, the main board is configured to adaptively control the ultrasonic drive signal and the high-frequency electric drive signal, so as to adaptively adjust at least one of the following output parameters: power of the ultrasonic drive signal, frequency of the ultrasonic drive signal, power of the high-frequency electric drive signal, and frequency of the high-frequency electric drive signal.
According to an embodiment of the present disclosure, the ultrasonic energy circuit board is provided with an ultrasonic signal collection circuit, comprising a first filter module, a first differential amplification module, a second filter module, a first automatic gain control module and a first analog-to-digital conversion module, configured to collect and process signals in a connection circuit between the ultrasonic energy circuit board and the surgical instrument to obtain an ultrasonic feedback signal, and provide the ultrasonic feedback signals to the main board, wherein the ultrasonic feedback signals includes an ultrasonic voltage feedback signal and an ultrasonic current feedback signal.
According to an embodiment of the present disclosure, the high-frequency electric energy circuit board is provided with a high-frequency electric acquisition circuit, including a third filter module, a second differential amplification module, a fourth filter module, a second automatic gain control module and a second analog-to-digital conversion module, configured to collect and process signals in a connection circuit of a high-frequency electrical energy circuit board and the surgical instrument, so as to obtain a high-frequency electric feedback signal; and provide the high-frequency electric feedback signal to the main board, wherein the high-frequency electric feedback signal includes a high-frequency voltage feedback signal and a high-frequency current feedback signal.

According to an embodiment of the present disclosure, the main board is configured to control the output parameter based on the ultrasonic feedback signal and the high-frequency electric feedback signal.

According to an embodiment of the present disclosure, the main board is further configured to obtain an acoustic impedance based on the ultrasonic feedback signal, obtain an electrical impedance based on the high-frequency electric feedback signal, match the acoustic impedance and/or the electrical impedance with impedance data to determine a tissue type, and determine the power of the ultrasonic drive signal and the power of the high-frequency electric drive signal based on the tissue type.

According to an embodiment of the present disclosure, the main board is further configured to obtain a first phase difference based on the ultrasonic voltage feedback signal and the ultrasonic current feedback signal; obtaining a second phase difference based on the high-frequency voltage feedback signal and the high-frequency current feedback signal; and determine the power of the ultrasonic drive signal and the power of the high-frequency electric drive signal based on the tissue type, the first phase difference, and the second phase difference.

According to an embodiment of the present disclosure, the main board is configured to determine a cutting stage based on the ultrasonic feedback signal and/or the high-frequency electric feedback signal, and determine the power of the ultrasonic drive signal and the power of the high-frequency electric drive signal based on the cutting stage.

According to an embodiment of the present disclosure, the ultrasonic energy circuit board includes a first network area and a first application area, the high-frequency electric energy circuit board includes a second network area and a second application area, and the first network area and the second network area are located close to a side of the main board.

According to an embodiment of the present disclosure, the ultrasonic energy circuit board is provided with at least one first element crossing the first network area and the first application area the first element is located between a pin of the first network area and a pin of the first application area, and the ultrasonic energy circuit board is provided with a first gap.

According to an embodiment of the present disclosure, the high-frequency electric energy circuit board is provided with at least one second element crossing the second network power area and the second application area, the second element is located at a position between a pin of the second network power area and a pin of the second application area, and the high-frequency electric energy circuit board is provided with a second gap.

According to an embodiment of the present disclosure, a distance between any two of the ultrasonic energy circuit board, the high-frequency electric energy circuit board, the main board, the power supply module and the power supply circuit board is not less than 10mm.

According to an embodiment of the present disclosure, a distance between each of the ultrasonic energy circuit board, the high-frequency electric energy circuit board, the main board, the power supply module and the power supply circuit board and the housing is not less than 10 mm.

According to an embodiment of the present disclosure, the first shield unit is the first shield enclosure disposed outside the ultrasonic energy circuit board, and a distance between the ultrasonic energy circuit board and a side wall of the first shield enclosure is not less than 10 mm.

According to an embodiment of the present disclosure, the second shield unit is the second shield enclosure disposed outside the high-frequency electric energy circuit board, and a distance between the high-frequency electric energy circuit board and a side wall of the second shield enclosure is not less than 10 mm.

According to an embodiment of the present disclosure, the housing is provided with a display screen, and the housing is provided with an interface, comprising one or more of a monopolar electric scalpel positive electrode interface, a monopolar electric scalpel negative electrode interface, a bipolar electric scalpel interface and an ultrasonic-electric scalpel interface.

According to an embodiment of the present disclosure, at the inner side of the housing, an insulating plate having a width of not less than 30 mm is provided at a position between the interface and the display screen.

According to an embodiment of the present disclosure, the device comprises a loudspeaker, which is arranged below the main board and is fixed on the housing by means of an adapter plate, a closed space is formed between an end face of the loudspeaker and a surface of the housing, and a sound outlet hole is provided at the bottom of the housing.

According to an embodiment of the present disclosure, the device further comprises a data interface and a download board, wherein the download board is connected to the main board and the data interface and is configured to receive the upgrade data transferred from the data interface and transfer the upgrade data to the main board.

According to an embodiment of the present disclosure, the power module set further includes a standby battery disposed below the power supply circuit board.

## Beneficial Effects of the Invention

### Beneficial Effects

According to the technical solutions of the embodiments of the present disclosure, the ultrasonic energy circuit board and the high-frequency electric energy circuit board are disposed in a stacking manner, and at least one of the ultrasonic energy circuit board and the high-frequency electric energy circuit board is isolated by the shielding unit, the interference of a high-frequency electric energy circuit board on an ultrasonic energy circuit board is effectively controlled, so that the high-frequency electric energy circuit board and the ultrasonic energy circuit board can be packaged in one output device, so that the interference of the high-frequency electric energy circuit board on the ultrasonic energy circuit board is effectively controlled, so that the ultrasonic energy circuit board and the high-frequency electric energy circuit board can be packaged in one output device, and the device can drive the ultrasonic surgical instrument and the high-frequency electrosurgical instrument at the same time, so that the equipment space is saved, the cost is reduced, and the user is also convenient to use.

## Brief Description of the Drawings

### Description of the Drawings

Other features, objects, and advantages of the disclosure will become more apparent from the following detailed description of non-limiting embodiments when taken in conjunction with the accompanying drawings. In the drawings:

Fig. 1 schematically illustrates a perspective view of an apparatus for outputting a drive signal to a surgical instrument according to an embodiment of the present disclosure;
Fig. 2 schematically illustrates a front view of an apparatus for outputting a drive signal to a surgical instrument according to an embodiment of the present disclosure;
Fig. 3 schematically illustrates a rear view of the device outputting a drive signal to a surgical instrument according to an embodiment of the present disclosure;
FIG. 4 schematically illustrates a bottom view of an apparatus for outputting a drive signal to a surgical instrument according to an embodiment of the present disclosure;
FIG. 5 schematically illustrates a cross-sectional view of an apparatus for outputting a drive signal to a surgical instrument according to an embodiment of the present disclosure;
FIG. 6 schematically illustrates a top view of the interior of the device outputting a drive signal to a surgical instrument according to an embodiment of the present disclosure;
FIG. 7 schematically illustrates a schematic view of a rear portion of a display screen of an apparatus for outputting a drive signal to a surgical instrument according to an embodiment of the present disclosure;
FIG. 8 schematically illustrates a second cross-sectional view of the device outputting a drive signal to a surgical instrument according to an embodiment of the present disclosure; and
FIG. 9 schematically illustrates a partial enlarged view of a second cross-sectional view at a speaker according to an embodiment of the present disclosure.

## Detailed Description of the Invention

### Detailed Description of the Embodiments

Hereinafter, exemplary embodiments of the present disclosure will be described in detail with reference to the accompanying drawings, so that those skilled in the art can easily implement them. In addition, for the sake of clarity, parts unrelated to the description of the exemplary embodiments are omitted in the accompanying drawings.

In the present disclosure, it should be understood that terms such as "include" or "have" are intended to indicate the presence of the features, numbers, steps, acts, components, parts, or combinations thereof disclosed in this specification and are not intended to exclude the possibility of one or more other features, numbers, steps, behaviors, components, parts, or combinations thereof present or added.

Where an expression similar to at least one of "A, B, and C, etc." is used, it should generally be understood by those skilled in the art that the meaning of the expression should include, but is not limited to, having A alone, independently having B, independently having C, having A and B, having A and C, systems having B and C, and/or having A, B, C, etc.). Where an expression similar to at least one of "A, B, or C, etc." is used, it should generally be understood by those skilled in the art that the meaning of the expression should include, but is not limited to, having A alone, independently having B, independently having C, having A and B, having A and C, systems having B and C, and/or having A, B, C, etc.).

In addition, it should be noted that, in the case of no conflict, the embodiments in the present disclosure and the features in the embodiments may be combined with each other. The present disclosure will be described in detail below with reference to the accompanying drawings and in combination with the embodiments.

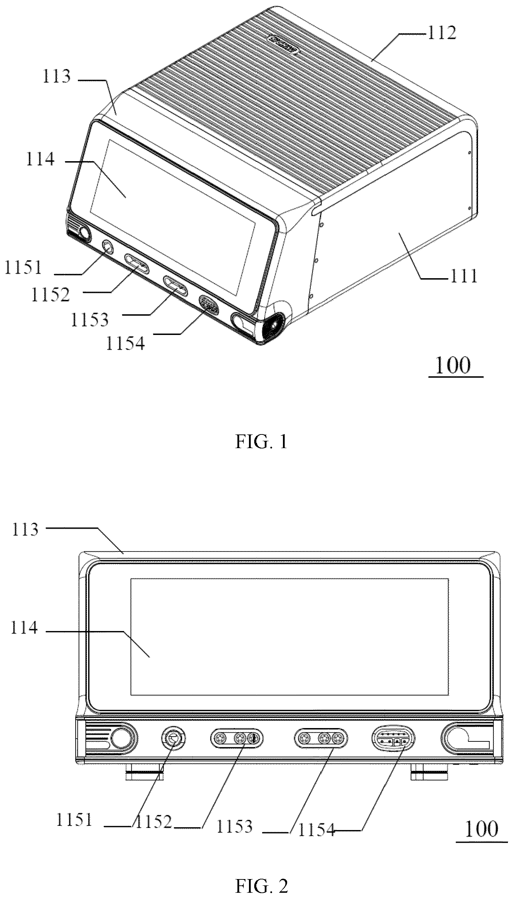

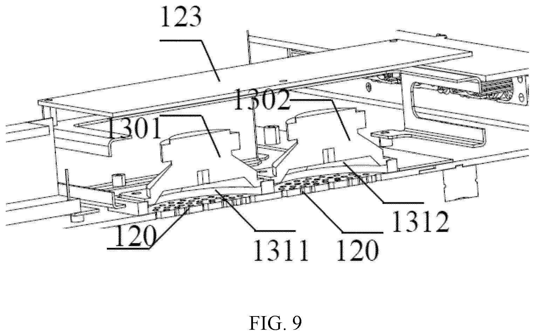

FIG. 1 to FIG. 9 schematically illustrate a schematic diagram of an apparatus 100 for outputting a drive signal to a surgical instrument according to an embodiment of the present disclosure. The following describes the device 100 in the embodiments of the present disclosure with reference to FIG. 1 to FIG. 9.

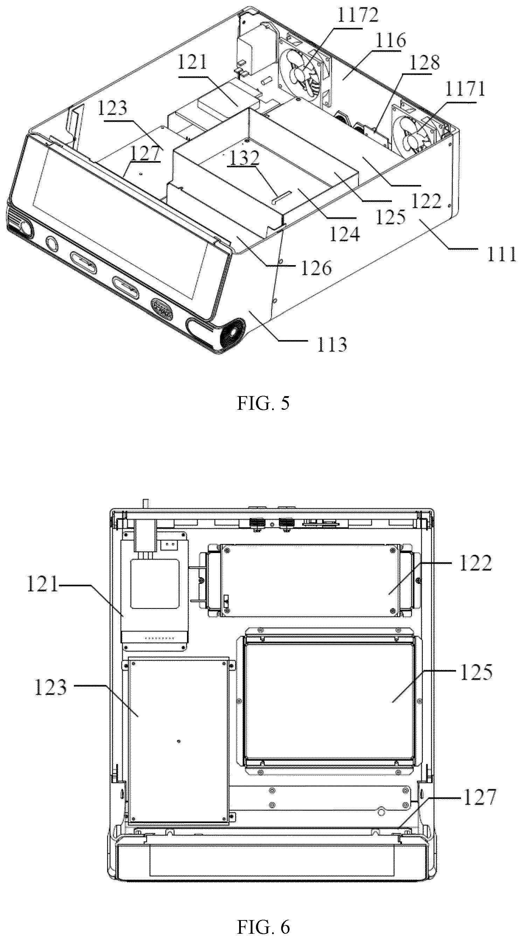

As shown in FIG. 5 and FIG. 6, the device 100 for outputting the drive signal to the surgical instrument includes a housing, and the housing is internally provided with a power module set, a main board 123, an ultrasonic energy circuit board 124, and a high-frequency electric energy circuit board.

The power supply module set includes a power supply module 121 and a power supply circuit board 122, wherein the power supply circuit board 122 is a PWR (Power Supply) board, and functions thereof include, but are not limited to, voltage adjustment, filtering, and the like. The power supply module set is configured to provide a stable power supply to the system.

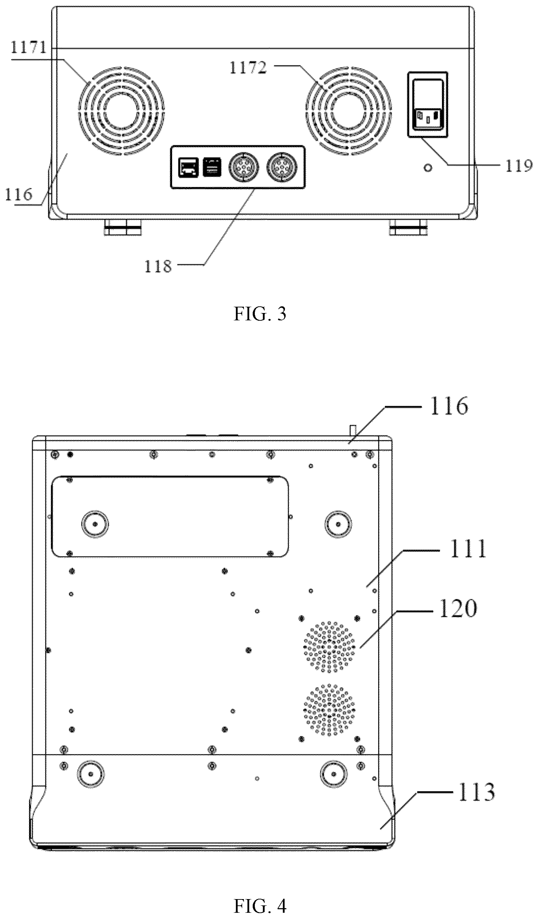

According to the embodiment of the present disclosure, as shown in Fig. 3, a power interface 119 may be disposed on the housing and configured to supply power to the power supply module 121. In some implementations of the present disclosure, the power supply module set can further include a standby battery, for example, disposed below the power supply circuit board 122, so that in an emergency situation, the surgery can be continue through the standby battery, thereby reducing the surgery risk.

The ultrasonic energy circuit board 124 is connected to the main board 123 and is configured to generate an ultrasonic drive signal. A shielding unit may be disposed outside the ultrasonic energy circuit board 124, such as the first shielding cover 125 shown in Fig. 5. It should be noted that, in order to clearly illustrate the ultrasonic energy circuit board 124, the top surface of the first shielding cover 125 is hidden in Fig. 5. The ultrasonic energy circuit board 124 can convert the power frequency alternating current into an ultrasonic electrical signal matching the ultrasonic transducer, and drive the ultrasonic transducer to convert electrical energy into mechanical energy, so as to drive the blade of the ultrasonic scalpel to vibrate. The operating frequency of a ultrasonic scalpel used by surgery is 20 ~ 100 kHz, which is most common at 55.5 kHz. The signal frequency output by the ultrasonic energy circuit board 124 in the embodiment of the present disclosure is also within this range.

The high-frequency electric energy circuit board is connected to the main board 123, and is configured to generate a high-frequency electric drive signal. A shielding unit may be disposed outside the high-frequency electric energy circuit board, such as the second shielding cover 126 shown in Fig. 5. The frequency range of a high-frequency electrosurgical instrument used for surgery is between 0.3 - 5 MHz. The term "high-frequency" in the present disclosure also refers to a frequency between 0.3- 5 MHz. In the embodiments of the present disclosure, the frequency of the drive signal output by the high-frequency electric energy circuit board also within this range.

According to an embodiment of the present disclosure, the ultrasonic energy circuit board 124 and the high-frequency electric energy circuit board may be disposed in a stacking manner, so that projections of the ultrasonic energy circuit board 124 and the high-frequency electric energy circuit board on the bottom surface of the housing at least partially overlap. As shown in Fig. 5, the ultrasonic energy circuit board 124 is disposed above the high-frequency electric energy circuit board, and the first shielding cover125 and the second shielding cover 126 may be separately disposed outside the two circuit boards. The first shielding cover 125 and the second shielding cover 126 are made of metal materials, so as to isolate interference between the ultrasonic energy circuit board 124 and the high-frequency electric energy circuit board, and interference between the ultrasonic energy circuit board 124 and the high-frequency electric energy circuit board and the outside world.

According to an embodiment of the present disclosure, the shielding unit may also be implemented as a magnetic bead or a magnetic ring. For example, a magnetic bead may be disposed on the input line of the ultrasonic energy circuit board 124 and connected in series in the line, so as to reduce unstable components in an input signal, thereby at least partially shielding external interference on the ultrasonic energy circuit board 124. Alternatively, a magnetic ring may be disposed, so that an input line of the ultrasonic energy circuit board 124 passes through the magnetic ring, which may also reduce unstable components in an input signal, thereby at least partially shielding external interference on the ultrasonic energy circuit board 124. Similarly, the high-frequency electric energy plate can be shielded in a similar manner, and of course, the shielding unit in the embodiment of the present disclosure can also be implemented by a combination of two or more of a shielding cover, a magnetic bead, or a magnetic ring.

According to the technical solutions of the embodiments of the present disclosure, the ultrasonic energy circuit board and the high-frequency electric energy circuit board are disposed in a stacking manner, and at least one of the ultrasonic energy circuit board and the high-frequency electric energy circuit board is isolated by the shielding unit, so that the interference of the high-frequency electric energy circuit board on the ultrasonic energy circuit board is effectively controlled, so that the high-frequency electric energy circuit board and the ultrasonic energy circuit board can be packaged in one output device. The device can simultaneously drive an ultrasonic surgical instrument and a high-frequency electrosurgical instrument, thereby saving the space of the device, facilitating the reduction of costs. It is also convenient for users to use.

The housing may have a variety of designs. In some embodiments of the present disclosure, as shown in Fig 1 to Fig. 5, the housing may include a U-shaped base 111, a back plate 116, a top cover 112 and a front cover 113. The housing may be made of an aluminum alloy material, to reduce the external impact of the device and the interference of the external environment to the device.

According to the embodiment of the present disclosure, the dimensions of the housing may satisfy a ratio of length, width and height of 1~3:1~3:1, for example, about 2:2: 1, so that the device 100 can be stably placed on the device table. The height of the housing may be 150-350 mm, and the size of the overall device may be, for example, 450mm×400 mm×200 mm, which is light and portable.

According to the embodiments of the present disclosure, a distance between any two of the ultrasonic energy circuit board 124, the high-frequency electric energy circuit board, the main board 123, the power supply module 121 and the power supply circuit board 122 is not less than a first preset value; a distance between each of the ultrasonic energy circuit board 124, the high-frequency electric energy circuit board, the main board 123, the power supply module 121 and the power supply circuit board 122 and the housing is not less than a second preset value; the distance between the ultrasonic energy circuit board 124 and the side wall of the first shielding cover 125 is not less than a third preset value; a distance between the high-frequency electric energy circuit board and a side wall of the second shielding cover 126 is not less than a fourth preset value. For example, the first preset value, the second preset value, the third preset value, and the fourth preset value may be set to 10 mm, preferably 12 mm.

According to the technical solutions of the embodiments of the present disclosure, certain intervals are reserved between various circuit boards, between the circuit boards and the housing, and between the energy circuit board (the ultrasonic energy circuit board, the high-frequency energy circuit board) and the shielding cover, which can reduce interference between circuits.

According to an embodiment of the present disclosure, the ultrasonic energy circuit board 124 comprises a first network power area and a first application area, the high-frequency electric energy circuit board comprises a second network power area and a second application area, and the first network power area and the second network power area are located on one side close to the main board 123.

According to an embodiment of the present disclosure, the first network power area or the second network power area is an area connected to the power supply module set. The first application area or the second application area is an area connected to an output end of the device. On any circuit board of the ultrasonic energy circuit board or the high-frequency electric energy circuit board, the voltage of the application area is greater than the voltage of the network power area. By arranging the two network power areas in the same direction, the interference of the high-voltage portion of the application area to the low-voltage portion of the application area can be reduced, and at the same time, the two application areas are arranged at one side away from the main board, and the interference of the application area to the main board can also be reduced.

According to an embodiment of the present disclosure, the ultrasonic energy circuit board 124 is provided with at least one first element crossing the first network power area and the first application area, the first element is located at a position between the pin of the first network power area and the pin of the first application area, and the ultrasonic energy circuit board 124 is provided with a first gap. The electrical gap between the pin of the first component located in the first power grid area and the pin located in the first application area meets design requirements. In the embodiments of the present disclosure, the first gap is disposed between the two pins, which can further increase the creepage distance and reduce the risk of dielectric electrode integration. The above-mentioned two pins are two pins nearest to each other on the ultrasonic energy circuit board 124, and in the case of ensuring the safety of the described two pins, the other pins of the first power grid area and the other pins of the first application area are also necessarily safe.

According to an embodiment of the present disclosure, the high-frequency electric energy circuit board is provided with at least one second element crossing the second network area and the second application area, the second element is located at a position between a pin of the second network area and a pin of the second application area, and the high-frequency electric energy circuit board is provided with a second gap. The electrical gap between the pin of the second component located in the second network area and the pin located in the second application area has met design requirements. In the embodiments of the present disclosure, the second gap is disposed between the two pins, which can further increase the creepage distance and reduce the risk of dielectric electrode formation. The above-mentioned two pins are two pins nearest to each other on the high-frequency electric energy circuit board, and in the case of ensuring the safety of the described two pins, other pins of the second network power area and other pins of the second application area are also necessarily safe.

According to an embodiment of the present disclosure, the ultrasonic energy circuit board 124 is provided with an ultrasonic frequency regulation module and an ultrasonic power regulation module for adjusting the frequency and power of the signal output by the ultrasonic signal generator. A high-frequency electric energy circuit board is provided with a high-frequency electric frequency regulation module and a high-frequency electric power regulation module for regulating the frequency and power of a signal output by a high-frequency signal generator. The main board 123 may be configured to adaptively control the ultrasonic drive signal and the high-frequency electric drive signal, so as to adaptively adjust at least one of the following: the power of the ultrasonic drive signal, the frequency of the ultrasonic drive signal, the power of the high-frequency electric drive signal, and the frequency of the high-frequency electric drive signal.

According to an embodiment of the present disclosure, the device 100 may collect and process the ultrasonic drive signal and the high-frequency electric drive signal, for example, collect and process the ultrasonic drive signal and the high-frequency electric drive signal through a dedicated circuit on the ultrasonic energy circuit board 124 and the high-frequency electric energy circuit board, and transmit the obtained signal to the main board 123. The main board 123 may determine an adjustment parameter based on the signal, and control the outputs of the ultrasonic energy circuit board 124 and the high-frequency electric energy circuit board based on the adjustment parameter. For example, when the ultrasonic-electric scalpel is used to cut the liver, the vibration of the ultrasonic scalpel rod can achieve the cutting function, and the application of high-frequency electric energy to the electric jaws can assist coagulation, when it is detected that the impedance increases, the signal power for driving the ultrasonic scalpel also increases, so as to accelerate the ultrasonic scalpel rod to vibrate, When the impedance is increased, the signal power applied to the pliers is also increased, and the efficiency of coagulation or evaporation of water can be improved, in this way, by using the ultrasonic-electric scalpel, the cutting progress can be accelerated, bleeding can be reduced, and a better surgical effect can be obtained. For another example, when the small intestine is cut, since the tissue toughness is high, the cutting time is relatively long, and when the ultrasonic-electric scalpel is used, the signal power for driving the ultrasonic-electric scalpel can be increased to accelerate the cutting, and the signal power applied to the electric pliers mouth keeps an appropriate level to evaporate moisture in the tissue, so as to assist the cutting.

According to the technical solutions of the embodiments of the present disclosure, by adaptively controlling an ultrasonic drive signal and a high-frequency electric drive signal, the surgical process can be better matched, and the surgical effect with excellent blood coagulation effect and cutting performance is facilitated.

According to an embodiment of the present disclosure, the ultrasonic energy circuit board is provided with an ultrasonic signal collection circuit, comprising a first filter module, a first differential amplification module, a second filter module, a first automatic gain control module and a first analog-to-digital conversion module, the ultrasonic feedback module is used for collecting and processing a signal in a connection circuit of the ultrasonic energy circuit board and the surgical instrument, so as to obtain an ultrasonic feedback signal, and provide the ultrasonic feedback signal to the main board, wherein the ultrasonic feedback signals include an ultrasonic voltage feedback signal and an ultrasonic current feedback signal.

Wherein the first filter module is used for primarily filtering the collected ultrasonic signal; and the first differential amplification module is used for differentially amplifying the signal after being primarily filtered; the second filter module, configured to perform secondary filtering on the signal after the differential amplification; the first automatic gain control module, configured to perform gain control on the signal after the secondary filtering, so as to perform digital sampling; and the first analog-to-digital conversion module is configured to perform digital conversion on the processed signal.

According to an embodiment of the present disclosure, the high-frequency electric energy circuit board is provided with a high-frequency electric acquisition circuit, including a third filter module, a second differential amplification module, a fourth filter module, a second automatic gain control module and a second analog-to-digital conversion module, for collecting and processing signals in a connection circuit of a high-frequency electrical energy circuit board and the surgical instrument, so as to obtain a high-frequency electric feedback signal, and providing the high-frequency electric feedback signal to the main board, wherein the high-frequency electric feedback signals include a high-frequency voltage feedback signal and a high-frequency current feedback signal.

Wherein the third filter module is used for primarily filtering the collected high-frequency electric signal; the second differential amplification module is used for differentially amplifying the signal after being primarily filtered; the fourth filter module is configured to perform secondary filtering on the signal after the differential amplification; the second automatic gain control module is configured to perform gain control on the signal after the secondary filtering to facilitate digital sampling; and the second analog-to-digital conversion module is configured to perform digital conversion on the processed signals.

According to the embodiments of the present disclosure, the main board 123 is used for controlling the output parameters based on the ultrasonic feedback signal and the high-frequency electric feedback signal, so as to match the surgical procedure, which is beneficial to achieve a surgical effect with excellent blood coagulation effect and cutting performance;; at the same time, the ultrasonic feedback signal and the high-frequency electric feedback signal are acquired by means of respective independent collection and processing circuits, so that the main board 123 adaptively controls the signal. And this structure can reduce interference between the ultrasonic signal and the high-frequency signal.

According to an embodiment of the present disclosure, the main board 123 is further configured to obtain an acoustic impedance based on the ultrasonic feedback signal, obtain an electrical impedance based on the high-frequency electric feedback signal, match the acoustic impedance and/or the electrical impedance with impedance data to determine a tissue type, and determine the power of the ultrasonic drive signal and the power of the high-frequency electric drive signal based on the tissue type.

Technical personnel in this field knows that an impedance in a load circuit can be obtained from a voltage signal V(t) and a current signal I(t), i. e. $R(t) = V(t)/I(t)$.

In the embodiments of the present disclosure, the impedance Rus(t) in an ultrasonic circuit, which is simply referred to as acoustic impedance, can be obtained by means of a voltage signal output by an ultrasonic energy circuit board and a current signal in a load circuit; and the impedance $R_{ES}(t)$ in the high-frequency circuit, which is simply referred to as electrical impedance, can be obtained by means of a voltage signal output by a high-frequency energy circuit board and a current signal.

In accordance with embodiments of the present disclosure, acoustic impedance, electrical impedance are related to the load circuit, i. e., in relation to the impedance characteristics of the tissue being cut. As described above, the type of the cut tissue can be identified by detecting acoustic impedance and/or electrical impedance in a surgical cutting process or a change rule thereof, and as a basis for adjusting the power of a drive signal, so as to self-adaptive adjustment of the power of the drive signal, thereby to obtain a better surgical effect.

For example, a time window for identifying a tissue type may be set in an initial cutting stage, and the detected acoustic impedance and/or electrical impedance is compared with impedance characteristic values of different types of tissues, where the tissue type identified has the highest matching degree. Since the physical properties of different tissues are different, they will also have impedance differences during cutting. By processing and analysing experimental data and empirical data, differentiated acoustic and/or electrical impedances of different types of tissues at the initial cutting stage can be obtained. These results can be stored and used to match tissue types.

Exemplarily, in the initial cutting stage, the identified tissue type is numbered as type 1, and a relationship between power P1(n+1) of the ultrasonic drive signal and power P2(n+1) of the high-frequency electric drive signal at a subsequent moment corresponding to the tissue type and acoustic impedance Rus(n) and electrical impedance $R_{ES}(n)$ at a previous moment is as follows:

$$P1(n+1) = f1(R_{US}(n), R_{ES}(n)),$$

$$P2(n+1) = f2(R_{US}(n), R_{ES}(n)),$$

If the identified tissue type number is type 2, the relationship between the power P1(n+1) of the ultrasonic drive signal and the power P2(n+1) of the high-frequency electric drive signal at a later moment corresponding to the identified tissue type number and the acoustic impedance Rus(n) and the electrical impedance $R_{ES}(n)$ at a previous moment are respectively:

$$P1(n+1) = f3(R_{US}(n), RES(n)),$$

$$P2(n+1) = f4(R_{US}(n), RES(n)).$$

wherein f1, f2, f3, and f4 are functions, and may be empirical functions or trained neural network models, for example; $R_{US}(n)$ and $R_{ES}(n)$ are the acoustic impedance and the electrical impedance at time n, respectively. By means of the embodiments of the present disclosure, when different tissues are encountered during surgical cutting, the cutting power more suitable for the tissues can also be automatically adjusted, thereby improving the cutting performance.

According to an embodiment of the present disclosure, the main board 123 is further configured to obtain a first phase difference based on the ultrasonic voltage feedback signal and the ultrasonic current feedback signal; obtaining a second phase difference based on the high-frequency voltage feedback signal and the high-frequency current feedback signal; and determine the power of the ultrasonic drive signal and the power of the high-frequency electric drive signal based on the tissue type, the first phase difference, and the second phase difference.

According to the phase difference between the voltage signal and the current signal in the load circuit, the power of the signal applied to the load circuit can be adjusted accurately. For example, the actual signal power loaded on the surgical instrument is $P=UIcos\theta$, and when the phase difference $\theta$ between the voltage signal and the current signal is not zero, the power of the actual signal will decrease. According to the embodiments of the present disclosure, after the tissue type is matched according to the impedance, if the phase difference in the current circuit is not zero, the signal power output by the energy source needs to be increased, so as to ensure that the signal power actually loaded on the surgical instrument satisfies requirements. For example, the currently matched tissue type is type 1, the corresponding power suggested value of the ultrasonic signal is P01, and the power suggested value of the high-frequency electrical signal is P02, at this time, the calculated first phase difference $\theta_1$ is not zero, that is, the phase difference of the ultrasonic feedback signal is not zero, and the second phase difference $\theta_2$ is zero. Therefore, the main board 123 controls the signal power Pus output by the ultrasonic energy circuit board to be $P_{US} = P01 /cos \theta_1$, and the output signal power of the high-frequency electric energy circuit board is $P_{ES} = P02$.

According to the embodiment of the present disclosure, the main board 123 may also control the time lengths for the ultrasonic energy circuit board and the high-frequency electric energy circuit board to output signals. For example, when the ultrasonic-electric scalpel is applied to perform surgical cutting, within a certain period of time, the ultrasonic energy circuit board is controlled to output the drive signal, However, the high-frequency electric energy circuit board does not output, and at this time, the ultrasonic-electric scalpel only executes the cutting function of the "ultrasonic scalpel", and in another time period, controlling the high-frequency electric energy circuit board to output a drive signal, while the ultrasonic energy circuit board does not output, and at this time, the ultrasonic-electric scalpel only executes a blood coagulation function of the "electric scalpel", which helps to obtain a better surgical effect.

According to an embodiment of the present disclosure, the main board 123 is configured to determine a cutting stage based on the ultrasonic feedback signal and/or the high-frequency electric feedback signal, and determine the power of the ultrasonic drive signal and the power of the high-frequency electric drive signal based on the cutting stage.

According to an embodiment of the present disclosure, the process of cutting may be divided into a initial cutting stage, a cutting progress stage, and a final cutting stage. Adjusting the drive signal power in each stage to adapt to the cutting process may bring benefits to the cutting. For example, in the initial cutting stage, if the ultrasonic scalpel needs to quickly start to work, the power of the driving the ultrasonic scalpel may be output at a higher value, and the power of the electric scalpel only needs to be at a lower value; by the stage of cutting, the cutter head has deep into the tissue, and needs to cut and synchronously coagulate. At this time, the respective advantages of the ultrasonic scalpel and the electric scalpel need to be used, and therefore the drive signal power is output at 50% of the total power of the ultrasonic scalpel and the electric scalpel; by the final stage of cutting, cutting is to be completed, and the advantages of fast cutting of the ultrasonic scalpel are fully utilized. At this time, the signal power for driving the ultrasonic scalpel is also output at a higher value, and the signal power for driving the electric scalpel only needs to be at a lower value.

According to the embodiments of the present disclosure, the cutting stage may be determined by the acoustic impedance and/or the electrical impedance, so as to output an adaptive ultrasonic drive signal and a high-frequency electric drive signal in different cutting stages. Experiments have found that the stage of cutting is also represented by the impedance characteristic of the tissue, for example, when cutting starts, the impedance increases and then decreases, which is the initial stage of cutting, and then a relatively long and constant stage appears, which is the stage of cutting, and when the final stage of cutting is reached, the impedance increases rapidly. Therefore, when it is detected that the acoustic impedance or the electric impedance firstly falls and then smoothes within a set time window, it can be identified that a cutting progress phase is entered, and when it is detected that the acoustic impedance or the electric impedance presents a rapidly rising and changing trend within the set time window, it is identified that a cutting end phase is entered.

According to embodiments of the present disclosure, a fixed power is may be used in the initial cutting stage and the final cutting stage, and a dynamically adjusted power is used during the stages of cutting. Optionally, in the initial cutting stage, the signal power for driving the ultrasonic scalpel and the electric scalpel can be proportionally output according to the ultrasonic power setting value and the high-frequency power setting value; in the cutting stage, the signal power for driving the ultrasonic scalpel and the electric scalpel can be adaptively adjusted according to acoustic impedance and/or electric impedance; in the final cutting stage , the power of the drive signal is still proportionally output according to the ultrasonic power setting value and the high-frequency power setting value. In this way, the power adaptation efficiency can be improved, and a better surgical effect can be obtained.

According to embodiments of the present disclosure, as shown in FIG. 1, FIG. 2, FIG. 5, and FIG. 7, a display screen 114 is disposed on the housing, and the device 100 further comprises a display circuit board 127 disposed behind the display screen 114 and configured to process a display signal so that the display screen 114 displays information. Through the display screen, the user can more intuitively aware of the state of the device 100.

According to embodiments of the present disclosure, the distance between the display circuit board 127 and any one of the main board 123, the ultrasonic energy circuit board 124, the high-frequency electric energy circuit board, the power supply module 121 and the power supply circuit board 122 is not less than 10 mm or 12 mm, thereby reducing interference between the circuits.

According to an embodiment of the present disclosure, a physical key and/or a virtual key may be disposed on the housing. For example, a physical on-off key may be disposed on the front cover 113 to start or close the device 100. For another example, the display screen of the front cover 113 may be a touch display screen, and one or more virtual keys may be provided, so as to implement various control functions, such as power level setting and operation mode selection. In some embodiments of the present disclosure, a control signal may also be transmitted to the device 100 through the interface as an input to the device.

According to embodiments of the present disclosure, as shown in Fig. 1 and Fig. 2, the housing is provided with an interface 115, including one or more of a monopolar electric scalpel positive-electrode interface 1151, a monopolar electric scalpel negative-electrode interface 1152, a bipolar electric scalpel interface 1153 and an ultrasonic-electric scalpel interface 1154.

In a working mode of the monopolar electric scalpel, the monopolar electric scalpel positive-pole interface 1151 and the monopolar electric scalpel negative-pole interface 1152 can be connected to the monopolar electric scalpel, and a high-frequency electric drive signal is provided thereto. At this time, components such as the power source module, the main board and the high-frequency electric energy circuit board work, and the ultrasonic energy circuit board can not work.

In a working mode of the bipolar electric scalpel, the bipolar electric scalpel interface 1153 can be connected to the bipolar electric scalpel, and the high-frequency electric drive signal is provided thereto. At this time, components such as the power source module, the main board and the high-frequency electric energy circuit board work, and the ultrasonic energy circuit board can not work.

In a working mode of the ultrasonic scalpel, the ultrasonic-electric scalpel interface 1154 can be connected to the ultrasonic scalpel to provide an ultrasonic drive signal and/or a high-frequency electric drive signal thereto, and at this time, components such as a power module set, a main board, an ultrasonic energy circuit board and a high-frequency electric energy circuit board all work. If only the function of the ultrasonic scalpel is used, the high-frequency electric energy circuit board may not work. In the case in which only the function of the ultrasonic scalpel is used, the device 100 may be simultaneously connected to one of the monopolar electric scalpel or the bipolar electric scalpel, so as to drive one of the monopolar electric scalpel or bipolar electric scalpel to work simultaneously with the ultrasonic scalpel.

According to the technical solutions of the embodiments of the present disclosure, the device is provided with a plurality of interfaces, which can support a plurality of application modes, and suitable for the requirements of a surgery.

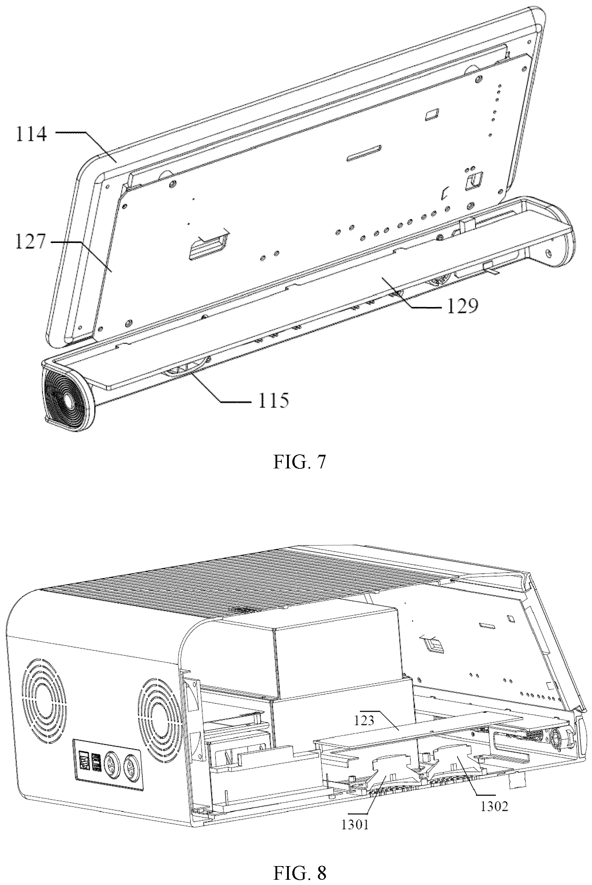

According to an embodiment of the present disclosure, as shown in Fig. 7, an insulating plate 129 having a width of not less than 30 mm is disposed at a position between the interface and the display screen 114 on the inner side of the housing. In the case of not interfering with other structural components and mounting components, the larger the width of the insulating plate 129 is, the better the insulation area is, thus increasing the creepage distance between the front side interface 115 and the display screen 114 and the display circuit board 127, thereby reducing the influence of the current at the interface on the display function and ensuring the electrical safety characteristics.

According to an embodiment of the present disclosure, as shown in Fig. 3 and Fig. 5, the device 100 further includes a data interface 118, which may be disposed on a rear side of the housing, for example, a USB interface or a web interface; a download board 128 connected to the main board 123 and the data interface 118 and configured to receive the upgrade data transferred from the data interface 118 and transfer the upgrade data to the main board 123. Because the device 100 generally has a high privacy requirement and is not suitable for maintaining and updating a program on the main board 123 through a network, the device 100 may be provided with the data interface 118, and when maintenance and update are required, upgrade data may be imported into the device 100 through a wired connection. The configuration of the download carrier is not only beneficial for importing the upgrade data, but also has a good line fixing effect.

According to embodiments of the present disclosure, as shown in Fig. 3 and Fig. 5, a plurality of fans 1171, 1172 may be provided on the housing, for example, may be disposed on the backplane. Two fans of the plurality of fans are in opposite directions, and at least comprise one fan blowing air to an inner side and one fan blowing air to an outer side, so as to form a stable air flow, thereby effectively improving the heat dissipation efficiency of the device.

According to embodiments of the present disclosure, as shown in Fig. 4, Fig. 8 and Fig. 9, the device 100 further includes loudspeakers 1301, 1302, which are arranged below the main board 123 and are fixed on the casing via adapter plates 1311, 1312, wherein a closed space is formed between an end face of the loudspeaker and a surface of the casing, and a sound outlet hole 120 is provided at the bottom of the housing.

According to an embodiment of the present disclosure, the adapter plate 1311 is fixed on the bottom surface of the housing and matches with the sound-outputting holes 120. The loudspeaker 1301 is fixed on the adapter plate 1311. The adapter plate 1311 is provided with a reserved hole with a size similar to that of the sound-emitting end face of the loudspeaker 1301. The sound-emitting end face of the loudspeaker 1301 is opposite to the sound-emitting hole of the housing through the reserved hole, so that the sound emitted by the loudspeaker 1301 can be emitted from the sound-emitting hole 120. The loudspeaker 1302 may be arranged in a manner similar to that of the loudspeaker 1301, and details are not repeatedly described herein. The material of the adapter plate can be an aluminium alloy material.

According to the technical solutions of the embodiments of the present disclosure, the adapter plate not only achieves the effect of fixing, but also achieves the effect of gathering sounds, and can improve the effect of sound playing.

The foregoing description is merely illustrative of the preferred embodiments of the present disclosure and of the technical principles applied thereto, as will be appreciated by those skilled in the art, the scope of the present disclosure is not limited to the technical solution formed by the specific combination of the described technical features, The present invention should also cover other technical solutions formed by any combination of the above technical features or their equivalent features without departing from the concept of the present invention. For example, the above features and technical features having similar functions disclosed in the present disclosure (but not limited to) are replaced with each other to form a technical solution.

**Claims**

1. A device for outputting drive signals to surgical instrument, comprising a housing, wherein the housing is internally provided with a power supply module set comprising a power supply module and a power supply circuit board, and the power supply module is configured to provide a power supply;

a main board;
an ultrasonic energy circuit board connected to the main board and used for generating an ultrasonic drive signal;
and a high-frequency electric energy circuit board connected to the main board and used for generating a high-frequency electric drive signal;
wherein projections of the ultrasonic energy circuit board and the high-frequency energy circuit board on a bottom face of the housing at least partially overlap, and a shielding unit is provided outside the ultrasonic energy circuit board and/or the high-frequency energy circuit board.

2. The device according to claim 1, wherein:

the ultrasonic energy circuit board is provided with an ultrasonic frequency regulation module and an ultrasonic power regulation module;
the high-frequency electric energy circuit board is provided with a high-frequency electric frequency regulation module and a high-frequency electric power regulation module;
the main board is configured to adaptively control the ultrasonic drive signal and the high-frequency electric drive signal, so as to adaptively adjust at least one of the following output parameters: power of the ultrasonic drive signal, frequency of the ultrasonic drive signal, power of the high-frequency electric drive signal, and frequency of the high-frequency electric drive signal.

3. The device according to claim 2, wherein:

the ultrasonic energy circuit board is provided with an ultrasonic signal collection circuit, comprising a first filter module, a first differential amplification module, a second filter module, a first automatic gain control module and a first analog-to-digital conversion module, configured to collect and process signals in a connection circuit between the ultrasonic energy circuit board and the surgical instrument to obtain an ultrasonic feedback signal, and provide the ultrasonic feedback signals to the main board, wherein the ultrasonic feedback signals includes an ultrasonic voltage feedback signal and an ultrasonic current feedback signal;
the high-frequency electric energy circuit board is provided with a high-frequency electric acquisition circuit, including a third filter module, a second differential amplification module, a fourth filter module, a second automatic gain control module and a second analog-to-digital conversion module, configured to collect and process signals in a connection circuit of a high-frequency electrical energy circuit board and the surgical instrument, so as to obtain a high-frequency electric feedback signal; and provide the high-frequency electric feedback signal to the main board, wherein the high-frequency electric feedback signal includes a high-frequency voltage feedback signal and a high-frequency current feedback signal;
and the main board is configured to control the output parameter based on the ultrasonic feedback signal and the high-frequency electric feedback signal.

4. The device according to claim 3, wherein the main board is further configured to obtain an acoustic impedance based on the ultrasonic feedback signal, obtain an electrical impedance based on the high-frequency electric feedback signal, match the acoustic impedance and/or the electrical impedance with impedance data to determine a tissue type, and determine the power of the ultrasonic drive signal and the power of the high-frequency electric drive signal based on the tissue type.

5. The device according to claim 4, wherein the main board is further configured to obtain a first phase difference based on the ultrasonic voltage feedback signal and the ultrasonic current feedback signal; obtaining a second phase difference based on the high-frequency voltage feedback signal and the high-frequency current feedback signal; and determine the power of the ultrasonic drive signal and the power of the high-frequency electric drive signal based on the tissue type, the first phase difference, and the second phase difference.

6. The device according to claim 3, wherein the main board is configured to determine a cutting stage based on the ultrasonic feedback signal and/or the high-frequency electric feedback signal, and determine the power of the ultrasonic drive signal and the power of the high-frequency electric drive signal based on the cutting stage.

7. The device according to any one of claims 1-6, wherein,

the ultrasonic energy circuit board includes a first network area and a first application area, the high-frequency electric energy circuit board includes a second network area and a second application area, and the first network area and the second network area are located close to a side of the main board;

the ultrasonic energy circuit board is provided with at least one first element crossing the first network area and the first application area the first element is located between a pin of the first network area and a pin of the first application area, and the ultrasonic energy circuit board is provided with a first gap;

the high-frequency electric energy circuit board is provided with at least one second element crossing the second network area and the second application area, the second element is located at a position between a pin of the second network area and a pin of the second application area, and the high-frequency electric energy circuit board is provided with a second gap.

8. The device according to any one of claims 1-6, wherein the device satisfies at least one of the following:

a distance between any two of the ultrasonic energy circuit board, the high-frequency electric energy circuit board, the main board, the power supply module and the power supply circuit board is not less than 10 mm;

a distance between each of the ultrasonic energy circuit board, the high-frequency electric energy circuit board, the main board, the power supply module and the power supply circuit board and the housing is not less than 10 mm;

the first shield unit is a first shield enclosure disposed outside the ultrasonic energy circuit board, and a distance between the ultrasonic energy circuit board and a side wall of the first shield enclosure is not less than 10 mm;

the second shield unit is a second shield enclosure disposed outside the high-frequency electric energy circuit board, and a distance between the high-frequency electric energy circuit board and a side wall of the second shield enclosure is not less than 10 mm.

9. The device according to any one of claims 1-6, wherein the housing is provided with a display screen, and the housing is provided with an interface, comprising one or more of a monopolar electric scalpel positive electrode interface, a monopolar electric scalpel negative electrode interface, a bipolar electric scalpel interface and an ultrasonic-electric scalpel interface;

and at the inner side of the housing, an insulating plate having a width of not less than 30 mm is provided at a position between the interface and the display screen.

10. The device according to any one of claims 1-6, wherein the device further has at least one of the following features:

the device comprises a loudspeaker, which is arranged below the main board and is fixed on the housing by means of an adapter plate, a closed space is formed between an end face of the loudspeaker and a surface of the housing, and a sound outlet hole is provided at the bottom of the housing;

the device further comprises a data interface and a download board, wherein the download board is connected to the main board and the data interface and is configured to receive the upgrade data transferred from the data interface and transfer the upgrade data to the main board;

and the power module set further includes a standby battery disposed below the power supply circuit board.

FIG. 1

FIG. 2

FIG. 3

FIG. 4

FIG. 5

FIG. 6

FIG. 7

FIG. 8

FIG. 9

## INTERNATIONAL SEARCH REPORT

| International application No. |
| --- |
| **PCT/CN2022/141414** |

### A. CLASSIFICATION OF SUBJECT MATTER

A61B17/32;A61B18/12

According to International Patent Classification (IPC) or to both national classification and IPC

### B. FIELDS SEARCHED

Minimum documentation searched (classification system followed by classification symbols)

IPC:A61B

Documentation searched other than minimum documentation to the extent that such documents are included in the fields searched

Electronic data base consulted during the international search (name of data base and, where practicable, search terms used)

CNTXT; ENTXTC; ENTXT; VEN; CNKI: 电路, 元件, 器件, 模块, 模组, 重叠, 层叠, 叠放, 叠置, 覆盖, 交错, 交叠, 重合, 错位, 屏蔽, 干扰, 隔离, 相位, 组织, 切割, 阶段, 引脚, 管脚, 端口, 接脚, 焊脚, 插脚, 击穿, 爬电, 安全, 短路, 基板, 控制板, 主板, 电路板, 芯片, 缝隙, 裂隙, 间隙, 空隙, 孔隙, 裂缝, 间隔, 槽, circuit, element, device, module, overlap, stack, overlay, cover +, interleave, shield, interference, isolat+, phase, tissue, stage, pin, foot, creepage, security, substrate, board, chip, slit, slot, gap

### C. DOCUMENTS CONSIDERED TO BE RELEVANT

| Category* | Citation of document, with indication, where appropriate, of the relevant passages | Relevant to claim No. |
| --- | --- | --- |
| PX | CN 114027937 A (SHANGHAI YICHAO MEDICAL DEVICES CO., LTD.) 11 February 2022 (2022-02-11) claims 1-10, description, paragraphs [0001]-[0099], and figures 1-9 | 1-10 |
| PX | CN 216876510 U (SHANGHAI YICHAO MEDICAL DEVICES CO., LTD.) 05 July 2022 (2022-07-05) claims 1-10, description, paragraphs [0001]-[0099], and figures 1-9 | 1-10 |
| Y | US 2018042659 A1 (COVIDIEN LP) 15 February 2018 (2018-02-15) description, paragraphs [0021]-[0036], and figures 1-4 | 1-10 |
| Y | CN 204734502 U (WUXI HISKY MEDICAL CO., LTD.) 04 November 2015 (2015-11-04) description, paragraph [0039] | 1-10 |
| Y | CN 109547220 A (SHENZHEN H&T INTELLIGENT CONTROL CO., LTD.) 29 March 2019 (2019-03-29) description, paragraphs [0026]-[0035], and figures 2-4 | 7 |

☑ Further documents are listed in the continuation of Box C.  ☑ See patent family annex.

| | |
| --- | --- |
| * Special categories of cited documents: <br> "A" document defining the general state of the art which is not considered to be of particular relevance <br> "D" document cited by the applicant in the international application <br> "E" earlier application or patent but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |
| Date of the actual completion of the international search <br><br> **20 March 2023** | Date of mailing of the international search report <br><br> **21 March 2023** |
| Name and mailing address of the ISA/CN <br><br> **China National Intellectual Property Administration (ISA/CN)** <br> **China No. 6, Xitucheng Road, Jimenqiao, Haidian District, Beijing 100088** | Authorized officer |
| Facsimile No. **(86-10)62019451** | Telephone No. |

Form PCT/ISA/210 (second sheet) (July 2022)

<div align="center">**INTERNATIONAL SEARCH REPORT**</div>

| International application No. |
|---|
| **PCT/CN2022/141414** |

**C.    DOCUMENTS CONSIDERED TO BE RELEVANT**

| Category* | Citation of document, with indication, where appropriate, of the relevant passages | Relevant to claim No. |
|---|---|---|
| A | US 2020078120 A1 (ETHICON LLC.) 12 March 2020 (2020-03-12) <br> entire document | 1-10 |
| A | CN 107648751 A (ANGEL MEDICAL TECHNOLOGY (NANJING) CO., LTD.) 02 February 2018 (2018-02-02) <br> entire document | 1-10 |
| A | CN 110662500 A (ETHICON LLC.) 07 January 2020 (2020-01-07) <br> entire document | 1-10 |
| A | CN 113208697 A (SHANGHAI YICHAO MEDICAL DEVICES CO., LTD.) 06 August 2021 (2021-08-06) <br> entire document | 1-10 |
| A | CN 211934284 U (SOUTH CHINA UNIVERSITY OF TECHNOLOGY) 17 November 2020 (2020-11-17) <br> entire document | 1-10 |
| A | CN 110916762 A (GUANGZHOU YIHE MEDICAL TECHNOLOGY DEVELOPMENT CO., LTD.) 27 March 2020 (2020-03-27) <br> entire document | 1-10 |
| A | US 2017000542 A1 (ETHICON ENDO SURGERY LLC.) 05 January 2017 (2017-01-05) <br> entire document | 1-10 |

Form PCT/ISA/210 (second sheet) (July 2022)

| Patent document cited in search report | | | Publication date (day/month/year) | Patent family member(s) | | | Publication date (day/month/year) |
|---|---|---|---|---|---|---|---|
| CN | 114027937 | A | 11 February 2022 | None | | | |
| CN | 216876510 | U | 05 July 2022 | None | | | |
| US | 2018042659 | A1 | 15 February 2018 | US | 11006997 | B2 | 18 May 2021 |
| | | | | EP | 3281711 | A1 | 14 February 2018 |
| | | | | US | 2021267659 | A1 | 02 September 2021 |
| CN | 204734502 | U | 04 November 2015 | None | | | |
| CN | 109547220 | A | 29 March 2019 | None | | | |
| US | 2020078120 | A1 | 12 March 2020 | WO | 2020051481 | A1 | 12 March 2020 |
| | | | | WO | 2020051481 | A8 | 01 April 2021 |
| | | | | BR | 112021003280 | A2 | 18 May 2021 |
| | | | | EP | 3846716 | A1 | 14 July 2021 |
| | | | | JP | 2022500109 | A | 04 January 2022 |
| CN | 107648751 | A | 02 February 2018 | None | | | |
| CN | 110662500 | A | 07 January 2020 | EP | 3634269 | A1 | 15 April 2020 |
| | | | | EP | 3634269 | B1 | 02 March 2022 |
| | | | | WO | 2018217548 | A2 | 29 November 2018 |
| | | | | WO | 2018217548 | A3 | 07 February 2019 |
| | | | | JP | 2020520726 | A | 16 July 2020 |
| | | | | EP | 3634268 | A1 | 15 April 2020 |
| | | | | EP | 3634268 | B1 | 09 March 2022 |
| | | | | US | 2018333182 | A1 | 22 November 2018 |
| | | | | US | 11229473 | B2 | 25 January 2022 |
| | | | | MX | 2019013902 | A | 20 January 2020 |
| | | | | WO | 2018217547 | A1 | 29 November 2018 |
| | | | | US | 2018333189 | A1 | 22 November 2018 |
| | | | | US | 11229475 | B2 | 25 January 2022 |
| | | | | BR | 112019023653 | A2 | 26 May 2020 |
| | | | | US | 2018333188 | A1 | 22 November 2018 |
| | | | | US | 11229474 | B2 | 25 January 2022 |
| | | | | MX | 2019013877 | A | 20 January 2020 |
| | | | | US | 2022202466 | A1 | 30 June 2022 |
| | | | | JP | 2020520722 | A | 16 July 2020 |
| | | | | JP | 2020520719 | A | 16 July 2020 |
| | | | | BR | 112019023456 | A2 | 30 June 2020 |
| | | | | MX | 2019013888 | A | 20 January 2020 |
| | | | | EP | 3634267 | A1 | 15 April 2020 |
| | | | | EP | 3634267 | B1 | 30 March 2022 |
| | | | | US | 2018333185 | A1 | 22 November 2018 |
| | | | | US | 11278340 | B2 | 22 March 2022 |
| | | | | US | 2018333190 | A1 | 22 November 2018 |
| | | | | US | 11259856 | B2 | 01 March 2022 |
| | | | | EP | 3634270 | A1 | 15 April 2020 |
| | | | | EP | 3634270 | B1 | 23 June 2021 |
| | | | | EP | 3629953 | A1 | 08 April 2020 |
| | | | | EP | 3629953 | B1 | 09 March 2022 |
| | | | | JP | 2020520725 | A | 16 July 2020 |
| | | | | MX | 2019013903 | A | 21 January 2020 |
| | | | | EP | 3629954 | A2 | 08 April 2020 |
| | | | | EP | 3629954 | B1 | 28 September 2022 |

Form PCT/ISA/210 (patent family annex) (July 2022)

<div style="text-align:center">

**INTERNATIONAL SEARCH REPORT**
Information on patent family members

</div>

International application No.

**PCT/CN2022/141414**

| Patent document cited in search report | | | Publication date (day/month/year) | Patent family member(s) | | | Publication date (day/month/year) |
|---|---|---|---|---|---|---|---|
| | | | | US | 2022202465 | A1 | 30 June 2022 |
| | | | | WO | 2018217552 | A1 | 29 November 2018 |
| | | | | BR | 112019024345 | A2 | 16 June 2020 |
| | | | | BR | 112019024303 | A2 | 16 June 2020 |
| | | | | JP | 2020520720 | A | 16 July 2020 |
| | | | | MX | 2019013876 | A | 20 January 2020 |
| | | | | WO | 2018217551 | A1 | 29 November 2018 |
| | | | | MX | 2019013895 | A | 20 January 2020 |
| | | | | BR | 112019023459 | A2 | 30 June 2020 |
| | | | | JP | 2020520721 | A | 16 July 2020 |
| | | | | JP | 7159217 | B2 | 24 October 2022 |
| | | | | BR | 112019024297 | A2 | 16 June 2020 |
| | | | | US | 2018333187 | A1 | 22 November 2018 |
| | | | | US | 11266455 | B2 | 08 March 2022 |
| | | | | WO | 2018217550 | A1 | 29 November 2018 |
| | | | | WO | 2018217549 | A1 | 29 November 2018 |
| CN | 113208697 | A | 06 August 2021 | None | | | |
| CN | 211934284 | U | 17 November 2020 | None | | | |
| CN | 110916762 | A | 27 March 2020 | WO | 2021000540 | A1 | 07 January 2021 |
| US | 2017000542 | A1 | 05 January 2017 | US | 10898256 | B2 | 26 January 2021 |
| | | | | EP | 3316810 | A2 | 09 May 2018 |
| | | | | JP | 2018519918 | A | 26 July 2018 |
| | | | | JP | 6797847 | B2 | 09 December 2020 |
| | | | | BR | 112017028403 | A2 | 28 August 2018 |
| | | | | BR | 112017028403 | B1 | 20 December 2022 |
| | | | | WO | 2017003854 | A2 | 05 January 2017 |
| | | | | WO | 2017003854 | A3 | 02 March 2017 |
| | | | | MX | 2018000194 | A | 11 April 2018 |

Form PCT/ISA/210 (patent family annex) (July 2022)